# EUROPEAN PATENT SPECIFICATION

(11) **EP 2 036 990 B1**
(45) Date of publication and mention of the grant of the patent: **02.04.2014**
(21) Application number: 08015997.3
(22) Date of filing: 11.09.2008
(51) Int. Cl.: C12Q 1/68

(54) **Diagnostic test for susceptibility to B-Raf kinase inhibitors**
Diagnosetest auf Anfälligkeit gegen B-Raf-Kinase-Hemmer
Test de diagnostic pour la susceptibilité aux inhibiteurs de la kinase B-Raf

(30) Priority: 11.09.2007 US 993391 P
(43) Date of publication of application: 18.03.2009
(73) Proprietor: Roche Diagnostics GmbH, 68305 Mannheim (DE); F.Hoffmann-La Roche AG, 4070 Basel (CH)
(72) Inventor: Langland, Rachel, Oakland, CA 94601 (US); Sharp, Thad, Parsippany, NJ 07054 (US); Will, Stephen, Oakland, CA 94611 (US); Wu, Lin, Moraga, CA 94556 (US)

(56) References cited:
- WO-A-2004/029288
- WO-A-2004/080464
- WO-A-2005/047542
- WO-A-2005/059171
- WO-A2-2005/074350
- US-A1- 2007 020 657
- RACHEL LANGLAND, THAD SHARP, JAMES TSAI, MICKEY WILLIAMS AND LIN WU: "Development of a companion diagnostic test for inhibitors of V600E BRAF"[Online] 15 September 2006 (2006-09-15), XP002505912 Retrieved from the Internet: URL:http://aacrmeetingabstracts.org/cgi/co ntent/abstract/2006/2/A13> [retrieved on 2008-11-25]
- BENLLOCH SUSANA ET AL: "Detection of BRAF V600E mutation in colorectal cancer: Comparison of automatic sequencing and real-time chemistry methodology" JOURNAL OF MOLECULAR DIAGNOSTICS, vol. 8, no. 5, November 2006 (2006-11), pages 540-543, XP002505913 ISSN: 1525-1578
- JIN LONG ET AL: "BRAF mutation analysis in fine needle aspiration (FNA) cytology of the thyroid" DIAGNOSTIC MOLECULAR PATHOLOGY, NEW YORK, NY, US, vol. 15, no. 3, 1 September 2006 (2006-09-01), pages 136-143, XP008098751
- IKENOUE TSUNEO ET AL: "Rapid detection of mutations in the BRAF gene using real-time polymerase chain reaction and melting curve analysis." CANCER GENETICS AND CYTOGENETICS, vol. 149, no. 1, February 2004 (2004-02), pages 68-71, XP002505914 ISSN: 0165-4608
- JARRY A ET AL: "Real-time allele-specific amplification for sensitive detection of the BRAF mutation V600E" MOLECULAR AND CELLULAR PROBES, ACADEMIC PRESS, LONDON, GB, vol. 18, no. 5, 1 October 2004 (2004-10-01), pages 349-352, XP004523911 ISSN: 0890-8508
- "Plexxikon and Roche enter partnership to develop targeted cancer therapeutic medicine PLX4032"[Online] 4 October 2006 (2006-10-04), XP002505915 Retrieved from the Internet: URL:http://www.rocheusa.com/newsroom/curre nt/2006/pr2006100401.html> [retrieved on 2008-11-25]

### SEQUENCE LISTING

<110> Roche Diagnostics GmbH
   F. Hoffmann-La Roche AG
<120> DIAGNOSTIC TEST FOR SUSCEPTIBILITY TO
   B-RAF KINASE INHIBITORS
<130> 24003 EP-HS
<140> Not yet assigned
   <141> Not yet assigned
<150> US 60/993,391
   <151> 2007-09-11
<160> 24
<170> FastSEQ for Windows Version 4.0
<210> 1
   <211> 31
   <212> DNA
   <213> Artificial Sequence
<220>
   <223> synthetic BRAF_MU probe
<221> modified_base
   <222> (6)...(6)
   <223> n= deoxyinosine
<221> modified_base
   <222> (8)...(8)
   <223> n= deoxyinosine
<221> modified_base
   <222> (31)...(31)
   <223> a = a with 3'-phosphate
<400> 1
   ctacananaa atctcgatgg agtgggtccc a 31
<210> 2
   <211> 29
   <212> DNA
   <213> Artificial Sequence
<220>
   <223> synthetic BRAF_WT probe
<221> modified_base
   <222> (4)...(4)
   <223> n = deoxyinosine
<221> modified_base
   <222> (29)...(29)
   <223> a = a with 3'-phosphate
<400> 2
   acantgaaat ctcgatggag tgggtccca 29
<210> 3
   <211> 30
   <212> DNA
   <213> Artificial Sequence
<220>
   <223> synthetic BRAF_F1 primer
<400> 3
   cctcacagta aaaataggtg attttggtct 30
<210> 4
   <211> 27
   <212> DNA
   <213> Artificial Sequence
<220>
   <223> synthetic BRAF_R5 primer
<400> 4
   tagcctcaat tcttaccatc cacaaaa 27
<210> 5
   <211> 116
   <212> DNA
   <213> Artificial Sequence
<220>
   <223> synthetic B-Raf V600E amplicon sequence
<400> 5
   cctcacagta aaaataggtg attttggtct agctacagtg aaatctcgat ggagtgggtc 60
   ccatcagttt gaacagttgt ctggatccat tttgtggatg gtaagaattg aggcta     116
<210> 6
   <211> 116
   <212> DNA
   <213> Homo sapiens
<220>
   <223> Homo sapiens chromosome X corresponding sequence to B-Raf
<400> 6
   cctcacagtg gaaataggtg attttggtct agccacagtg aaatcttgat ggagtgggtc 60
   ccatcagttt gaacagttgt ctggatcttt tttgtgtatg gcaccagaag tcatca     116
<210> 7
   <211> 40
   <212> PRT
   <213> Homo sapiens
<220>
   <223> B-Raf exon 15 region surrounding codon 600
<400> 7
   Asn Ile Phe Leu His Glu Asp Leu Thr Val Lys Ile Gly Asp Phe Gly
   1               5                   10                  15
   Leu Ala Thr Val Lys Ser Arg Trp Ser Gly Ser His Gln Phe Glu Gln
               20                  25                  30
   Leu Ser Gly Ser Ile Leu Trp Met
           35                  40
<210> 8
   <211> 40
   <212> PRT
   <213> Homo sapiens
<220>
   <223> A-Raf region
<400> 8
   Asn Ile Phe Leu His Glu Gly Leu Thr Val Lys Ile Gly Asp Phe Gly
   1               5                   10                  15
   Leu Ala Thr Val Lys Thr Arg Trp Ser Gly Ala Gln Pro Leu Glu Gln
               20                  25                  30
   Pro Ser Gly Ser Val Leu Trp Met
           35                  40
<210> 9
   <211> 40
   <212> PRT
   <213> Homo sapiens
<220>
   <223> C-Raf region
<400> 9
   Asn Ile Phe Leu His Glu Gly Leu Thr Val Lys Ile Gly Asp Phe Gly
    1               5                  10                  15
   Leu Ala Thr Val Lys Ser Arg Trp Ser Gly Ser Gln Gln Val Glu Gln
               20                  25                  30
   Pro Thr Gly Ser Val Leu Trp Met
           35                  40
<210> 10
   <211> 116
   <212> DNA
   <213> Homo sapiens
<220>
   <223> RAF1; encodes C-Raf
<400> 10
   cttaacagtg aaaattggag attttggttt ggcaacagta aagtcacgct ggagtggttc 60
   tcagcaggtt gaacaaccta ctggctctgt cctctggatg gtgagaatct gggctc     116
<210> 11
   <211> 116
   <212> DNA
   <213> Homo sapiens
<220>
   <223> ARAF; encodes A-Raf
<400> 11
   gctcacggtg aagatcggtg actttggctt ggccacagtg aagactcgat ggagcggggc 60
   ccagcccttg gagcagccct caggatctgt gctgtggatg gtgagttggg ccaggc     116
<210> 12
   <211> 30
   <212> DNA
   <213> Artificial Sequence
<220>
   <223> synthetic BRaf reverse primer
<400> 12
   aaatagcctc aattcttacc atccacaaaa 30
<210> 13
   <211> 27
   <212> DNA
   <213> Artificial Sequence
<220>
   <223> synthetic BRaf reverse primer
<400> 13
   tagcctcaat tcttaccatc cacaaaa 27
<210> 14
   <211> 26
   <212> DNA
   <213> Artificial Sequence
<220>
   <223> synthetic BRaf reverse primer
<221> modified_base
   <222> (26)...(26)
   <223> a = a with 6-carboxyhexachlorofluorescein (HEX)
<400> 14
   tagcctcaat tcttaccatc cacaaa 26
<210> 15
   <211> 28
   <212> DNA
   <213> Artificial Sequence
<220>
   <223> synthetic BRaf reverse primer
<400> 15
   agggccaaaa atttaatcag tggaaaaa 28
<210> 16
   <211> 29
   <212> DNA
   <213> Artificial Sequence
<220>
   <223> synthetic BRaf reverse primer
<400> 16
   cagtggaaaa atagcctcaa ttcttacca 29
<210> 17
   <211> 29
   <212> DNA
   <213> Artificial Sequence
<220>
   <223> synthetic BRaf forward primer
<221> modified_base
   <222> (29)...(29)
   <223> t = t with 6-carboxyhexachlorofluorescein (HEX)
<400> 17
   tttcttcatg aagacctcac agtaaaaat 29
<210> 18
   <211> 30
   <212> DNA
   <213> Artificial Sequence
<220>
   <223> synthetic BRaf forward primer
<221> modified_base
   <222> (30)...(30)
   <223> a = a with 6-carboxyhexachlorofluorescein (HEX)
<400> 18
   atatatttct tcatgaagac ctcacagtaa 30
<210> 19
   <211> 23
   <212> DNA
   <213> Artificial Sequence
<220>
   <223> synthetic BRaf reverse primer
<400> 19
   atgacttctg gtgccatcca caa 23
<210> 20
   <211> 32
   <212> DNA
   <213> Artificial Sequence
<220>
   <223> synthetic BRaf reverse primer
<400> 20
   aaaaatagcc tcaattctta ccatccacaa aa 32
<210> 21
   <211> 25
   <212> DNA
   <213> Artificial Sequence
<220>
   <223> synthetic BRaf reverse primer
<400> 21
   gccatccaca aaatggatcc agaca 25
<210> 22
   <211> 27
   <212> DNA
   <213> Artificial Sequence
<220>
   <223> synthetic BRaf reverse primer
<400> 22
   caaaatggat ccagacaact gttcaaa 27
<210> 23
   <211> 24
   <212> DNA
   <213> Artificial Sequence
<220>
   <223> synthetic TTS155-BRAF_MU probe
<221> modified_base
   <222> (1)...(1)
   <223> n = deoxyinosine attached to 3' of deoxyribose phosphate backbone modified by FAM attached to 3' of oligonucleotide 5'CTACAIA3', where 5' C is modified by BHQ2 quencher and I = deoxyinosine
<221> modified_base
   <222> (24)...(24)
   <223> a = a with 3'-phosphate
<400> 23
   naaatctcga tggagtgggt ccca 24
<210> 24
   <211> 24
   <212> DNA
   <213> Artificial Sequence
<220>
   <223> synthetic TTS148-BRAF_WT probe
<221> modified_base
   <222> (1)...(1)
   <223> n = g attached to 3' of deoxyribose phosphate backbone modified by HEX attached to 3' of oligonucleotide 5'ACAIT3', where 5' A is modified by BHQ2 quencher and I = deoxyinosine
<221> modified_base
   <222> (24)...(24)
   <223> a = a with 3'-phosphate
<400> 24
   naaatctcga tggagtgggt ccca 24
<210> 25
   <211> 30
   <212> DNA
   <213> Artificial Sequence
<220>
   <223> Synthetic TTS068-BRAF_F1 Primer
<221> modified_base
   <222> (30)...(30)
   <223> t=t with t-butyl benzyl dA
<400> 30
   cctcacagta aaaataggtg attttggtct 30

## Description

### BACKGROUND OF THE INVENTION

The *BRAF* gene encodes B-Raf, a serine/threonine kinase, which couples signaling from activated RAS to downstream MAPK kinases (Wellbrock et al., Cancer Res. 64:2338-2342, 2004). Oncogenic mutations in B-Raf result in gain-of-kinase-function, rendering the Raf-MEK-ERK pathway constitutively active in the absence of the typical growth factors. This constitutive activation correlates with poor prognosis in metastatic melanoma (Houben *et al*., 2004, *supra*). Activating mutations in *BRAF* have been reported in a variety of malignancies. For example, mutations in *BRAF* are found in as many as 70% of human melanoma cases. A single-base mutation (T>A) at nucleotide position 1799 in codon 600 of exon 15 leads to a valine-to-glutamate substitution (V600E), which is present in more than 85% of melanomas with a mutation in B-Raf (Davies et al., Nature 417: 949-954, 2002; Garnett and Marais, Cancer Cell 6: 313-319, 2004; Gray-Schopfer et al., Cancer Metastasis Rev. 24:165-183, 2005; Houben et al., 2004). Less commonly, V600E results from the two-base mutation TG>AA at nucleotide positions 1799-1800 (Houben et al., J Carcinog. 3:6, 2004).

A number of kinase inhibitors are known, including those that inhibit B-Raf. Such inhibitors include PLX4032, which selectively inhibit B-Raf V600E kinase activity. The current invention provides methods of identifying V600E-positive patients to select for treatment using a B-Raf kinase inhibitor, *e.g*., PLX4032.

LANGLAND et al., 2006 ("Development of a companion diagnostic test for inhibitors of V600E BRAF", URL:http://aacrmeetingabstracts.org/cgi/content/abstract/2006/2/A13) discloses a method to determine the sensitivity of cancer cells to PLX4032 based on the detection of the *BRAF^{V600E}* mutation by real-time TaqMan PCR. However, no specific primers or probe sequences are disclosed.

### BRIEF SUMMARY OF THE INVENTION

The invention provides methods and kits for the detection of patients who are candidates for treatment with a B-Raf kinase inhibitor. Thus, in one aspect, the invention provides a method of determining sensitivity of cancer cells to a B-Raf inhibitor, the method comprising: (i) providing a nucleic acid sample from cancer cells from a patient that has a cancer; (ii) amplifying a target polynucleotide sequence in the nucleic acid sample using a primer pair that amplifies the target polynucleotide sequence, wherein the target polynucleotide sequence comprises a V600E, a V600D or a V600K mutation site in *BRAF* and amplification is performed in the presence of a labeled oligonucleotide probe that comprises the sequence set forth in SEQ ID NO:1 wherein "n" is deoxyinosine, and detects the presence of a mutated sequence at the V600E mutation site in BRAF; and (iii) detecting the presence or absence of a V600E, or V600D or V600K mutation in BRAF; thereby determining the sensitivity of the cancer to the B-Raf inhibitor. In some embodiments, the probe has the nucleotide sequence set forth in SEQ ID NO:1. The amplification can be performed in the presence of a second probe that detects the presence of a wild type sequence at the V600E mutation site. In some embodiments, the second probe comprises at least 15 nucleotides of SEQ ID NO:2. In some embodiments the second probe has the nucleotide sequence set forth in SEQ ID NO:2. In certain embodiments the probe can be labeled with a fluorescent label. The probe can also be labeled with two labels, where one of the labels is a fluorescent dye and the other label is a quenching moiety.

In some embodiments, both primers of the primer pair used in the amplification reaction hybridizes to exon 15 of *BRAF.* In some embodiments, one of the primers of the primer pair used in the amplification reactions comprises at least 15 contiguous nucleotides of the nucleotide sequence set forth in SEQ ID NO:3, *e.g*., one of the primers in the primer pair can have the nucleotide sequence set forth in SEQ ID NO:3. In further embodiments, one of the primers in the primer pairs comprises at t least 15 contiguous nucleotides of the nucleotide sequence set forth in SEQ ID NO:4. For example, the primer can have the nucleotide sequence set forth in SEQ ID NO:4. In further embodiments, the primer pair used for the amplification comprises primers having the nucleotide sequences set forth in SEQ ID NO:3 and SEQ ID NO:4.

In some embodiments, the step of amplifying the reaction comprises an RT-PCR.

The method can be employed to detect cancers that have a mutation at amino acid position 600 of B-Raf, *e.g*., a V600E mutation. In some embodiments, the cancer is melanoma. In other embodiments, the cancer is colon cancer or thyroid cancer. In some embodiments, the nucleic acid sample used in the methods of the invention to detect the mutation can be from a skin biopsy. In other embodiments, the sample is from a colon biopsy. The sample can also be from paraffin-embedded tissue. The method of the invention can further comprise recording a diagnosis that the patient is sensitive to a B-Raf inhibitor, such as a mutant-specific B-Raf inhibitor, *e.g*., PLX4032.

In some embodiments, of the disclosure the method further comprises administering a B-Raf inhibitor to the patient. The B-Raf inhibitor can be a mutant specific B-Raf inhibitor such as, PLX4032.

In another aspect, the disclosure provides a method of identifying a PLX4032 treatment candidate, the method comprising: providing a sample from a subject; and detecting a biomolecule comprising a *BRAF* V600E mutation from the sample, thereby identifying the PLX4032 treatment candidate. In some embodiments, the biomolecule is a nucleic acid. In other embodiments, the biomolecule is a polypeptide. The polypeptide can be obtained, *e.g*., from a sample comprising cancer cells from the patient. In some embodiments, the polypeptide may be detected using an immunoassay. The method can also comprise administering PLX4032 to the subject.

In another aspect, the invention provides a kit for detecting a patient that is a candidate for treatment with a B-Raf inhibitor, wherein the kit comprises a first allele-specific probe, wherein the first probe is suitable for detecting a V600E, a V600D or a V600K mutation in *BRAF* and comprises the sequence set forth in SEQ ID NO: 1. In some embodiments, probe has the nucleotide sequence set forth in SEQ ID NO:1. A kit of the invention may further comprise a second allele-specific probe, wherein the second probe detects the wild type *BRAF* sequence and comprises at least 15 contiguous of the nucleotide sequence set forth in SEQ ID NO:2. In some embodiments, the second probe has the nucleotide sequence set forth in SEQ ID NO:2.

In further embodiments, a kit of the invention further comprises a primer pair that amplifies a target region of *BRAF* that comprises a V600E mutation site. For example, the primer pair can comprise a primer that comprises at least 15 contiguous nucleotides of the nucleotide sequence set forth in SEQ ID NO:3. In certain aspect the primer has the nucleotide sequence set forth in SEQ ID NO:3. In other embodiments the primer pair can comprise primers that comprise at least 15 contiguous nucleotides of the nucleotide sequence set forth in SEQ ID NO:3 and SEQ ID NO:4. In some embodiments, the primer pair comprises primers that have the nucleotide sequences set forth in SEQ ID NO:3 and SEQ ID NO:4.

Thus, in some embodiments, a kit of the invention can comprise: a probe that has the nucleotide sequence set forth in SEQ ID NO:1; a probe that has the nucleotide sequence set forth in SEQ ID NO:2; a primer that has the nucleotide sequence set forth in SEQ ID NO:3; and a primer that has the nucleotide sequence set forth in SEQ ID NO:4.

### BRIEF DESCRIPTION OF THE DRAWINGS

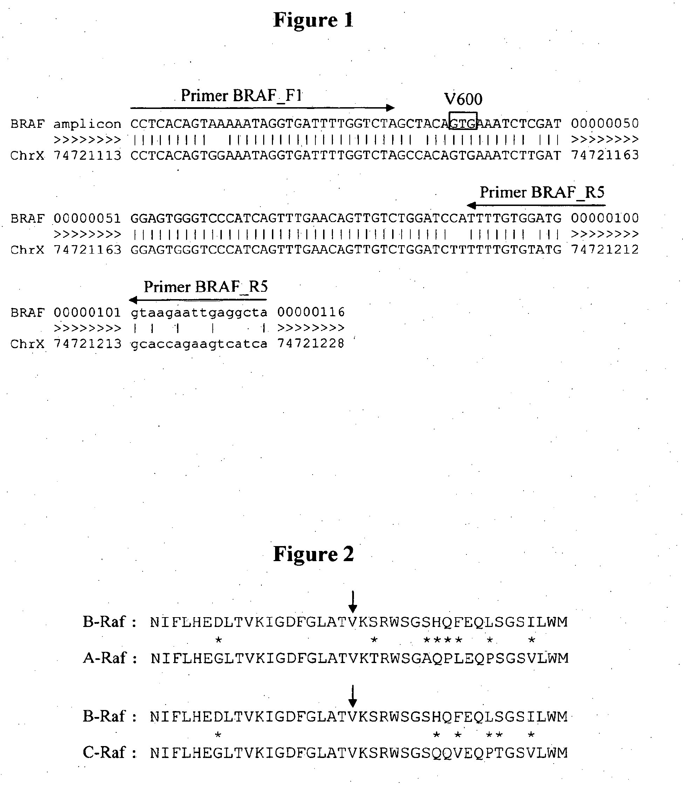

Figure 1 shows an alignment of a B-Raf V600E amplicon (SEQ ID NO:5) with a corresponding region of Chromosome X (SEQ ID NO:6). The B-Raf V600E primer sites are shown with arrows. The amplicon includes portions of exon 15 (uppercase) and intron 15 (lowercase). Vertical bars denote positions of identity between the *BRAF* and chromosome X sequences. Codon 600 is boxed; GTG corresponds to valine (V). The probe binding region is highlighted by shading; the mutant (MU) probe is longer than the wild type (WT) probe by two nucleotides (5'-CT in highlighted region) and both probes bind to the complement of the sequence shown here.
Figure 2 shows an alignment of a B-Raf exon 15 region surrounding codon 600 (arrow) (SEQ ID NO:7) with homologous regions of A-Raf (SEQ ID NO:8) and C-Raf (SEQ ID NO:9). Asterisks mark amino acid differences relative to the B-Raf sequence (*e.g*., Mercer & Pritchard, Biochim Biophys Acta 1653:25-40,2003).

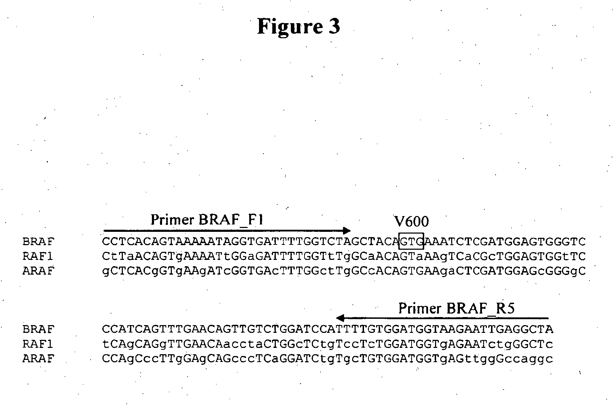

Figure 3 shows an alignment of the a B-Raf V600E amplicon (SEQ ID NO:5) with the corresponding sequences from the *ARAF* (encodes A-Raf) (SEQ ID NO:11) and *RAF1* (encodes C-Raf) (SEQ ID NO:10) genes. Nucleotides in lowercase differ from the *BRAF* sequence.

### DETAILED DESCRIPTION OF THE INVENTION

### Definitions

In the context of this application, a "V600E" refers to a mutation in *BRAF* (T>A at nucleotide position 1799) that results in substitution of a glutamine for a valine at amino acid position 600 of B-Raf. "V600E" is also known as "V599E" (1796T>A) under a previous numbering system (Kumar et al., Clin. Cancer Res. 9:3362-3368, 2003).

In the context of this invention, a "B-Raf kinase inhibitor" inhibits activity of a B-Raf kinase. Such an inhibitor may also inhibit the activity of other kinases, including other raf kinases.

A "mutant-specific B-Raf kinase inhibitor" as used herein refers to a B-Raf inhibitor that has selectivity for a mutant B-Raf such as B-Raf having a mutation at the valine residue at amino acid position 600, *e.g*., a V600E mutation, compared to wild type B-Raf. Such an inhibitor is at least two times, more often at least three times, or more as potent an inhibitor of mutant B-Raf, *e.g*., a B-Raf having a V600E mutation, in comparison to wild type. For example, in some embodiments, a "mutant-specific B-Raf inhibitor" may have an IC₅₀ for kinase inhibition activity (biochemical assay) of about 30 nM for V600E B-Raf whereas the corresponding IC₅₀ for wild type B-Raf is about 100 nM. The potency can also be compared in terms of IC₅₀ values for cellular assays, *e.g*., cellular assays that measure growth inhibition. A "mutant-specific B-Raf inhibitor" in the context of this invention may also inhibit kinases other than B-Raf, *e.g*., other raf kinases.

The term "hybridization" refers to the formation of a duplex structure by two single stranded nucleic acids due to complementary base pairing. Hybridization can occur between exactly complementary nucleic acid strands or between nucleic acid strands that contain minor regions of mismatch. As used herein, the term "substantially complementary" refers to sequences that are complementary except for minor regions of mismatch. Typically, the total number of mismatched nucleotides over a hybridizing region is not more than 3 nucleotides for sequences about 15 nucleotides in length. Conditions under which only exactly complementary nucleic acid strands will hybridize are referred to as "stringent" or "sequence-specific" hybridization conditions. Stable duplexes of substantially complementary nucleic acids can be achieved under less stringent hybridization conditions. Those skilled in the art of nucleic acid technology can determine duplex stability empirically considering a number of variables including, for example, the length and base pair concentration of the oligonucleotides, ionic strength, and incidence of mismatched base pairs. For example, computer software for calculating duplex stability is commercially available from National Biosciences, Inc. (Plymouth, Minn.); *e.g*., OLIGO version 5, or from DNA Software (Ann Arbor, Michigan), *e.g*., Visual OMP 6.

Stringent, sequence-specific hybridization conditions, under which an oligonucleotide will hybridize only to the exactly complementary target sequence, are well known in the art (see, e.g., the general references provided in the section on detecting polymorphisms in nucleic acid sequences). Stringent conditions are sequence-dependent and will be different in different circumstances. Generally, stringent conditions are selected to be about 5°C lower than the thermal melting point (Tm) for the specific sequence at a defined ionic strength and pH. The Tm is the temperature (under defined ionic strength and pH) at which 50% of the base pairs have dissociated. Relaxing the stringency of the hybridizing conditions will allow sequence mismatches to be tolerated; the degree of mismatch tolerated can be controlled by suitable adjustment of the hybridization conditions.

The term "primer" refers to an oligonucleotide that acts as a point of initiation of DNA synthesis under conditions in which synthesis of a primer extension product complementary to a nucleic acid strand is induced, i.e., in the presence of four different nucleoside triphosphates and an agent for polymerization (i.e., DNA polymerase or reverse transcriptase) in an appropriate buffer and at a suitable temperature. A primer is preferably a single-stranded oligodeoxyribonucleotide. The primer includes a "hybridizing region" exactly or substantially complementary to the target sequence, preferably about 15 to about 35 nucleotides in length. A primer oligonucleotide can either consist entirely of the hybridizing region or can contain additional features which allow for the detection, immobilization, or manipulation of the amplified product, but which do not alter the ability of the primer to serve as a starting reagent for DNA synthesis. For example, a nucleic acid sequence tail can be included at the 5' end of the primer that hybridizes to a capture oligonucleotide.

An "allele-specific" primer, as used herein, is a primer that hybridizes to a target sequence such that the 3' end, usually the 3' nucleotide, of the primer aligns with a site of interest, *e.g*., nucleotide 1799, which is the second position within codon 600 of *BRAF,* and is exactly complementary to either the wild type allele or mutant allele at the position of interest. As used herein, the primer is "specific for" the allele to which it is exactly complementary at the 3' end, *e.g*., at the 3' nucleotide or penultimate nucleotide. In general, primer extension is inhibited when a mismatch is present at the 3' end of the primer. An allele-specific primer, when hybridized to the exactly complementary allele, is extendable at a greater efficiency. The same primer, when hybridized to the other allele, is not as readily extendable because of the mismatch at the 3' end, *e.g*., the 3' nucleotide or penultimate nucleotide at the 3' end, of the primer in the hybridization duplex. Thus, the use of an allele-specific primer provides allelic discrimination based on differential formation of an extension product.

The term "probe" refers to an oligonucleotide that selectively hybridizes to a target nucleic acid under suitable conditions.

An "allele-specific" probe contains a "hybridizing region" exactly or substantially complementary to the target sequence, and is exactly complementary to the target sequence at the site of interest, *e.g*., nucleotide 1799 in codon 600 of *BRAF.* Thus, for example, a V600E allele-specific probe selectively detects a V600E mutation sequence, whereas a wild type *BRAF* allele-specific probe selectively detects the wild type sequence. A hybridization assay carried out using the probe under sufficiently stringent hybridization conditions enables the selective detection of a specific target sequence comprising the site of interest. The probe hybridizing region is preferably from about 10 to about 35 nucleotides in length, more preferably from about 15 to about 35 nucleotides in length. The use of modified bases or base analogues which affect the hybridization stability, which are well known in the art, may enable the use of shorter or longer probes with comparable stability. A probe oligonucleotide can either consist entirely of the hybridizing region or can contain additional features which allow for the detection or immobilization of the probe, but which do not significantly alter the hybridization characteristics of the hybridizing region.

The term "target sequence" or "target region" refers to a region of a nucleic acid that is to be analyzed and comprises the polymorphic site of interest.

As used herein, the terms "nucleic acid," "polynucleotide" and "oligonucleotide" refer to primers, probes, and oligomer fragments. The terms are not limited by length and are generic to linear polymers of polydeoxyribonucleotides (containing 2-deoxy-D-ribose), polyribonucleotides (containing D-ribose), and any other N-glycoside of a purine or pyrimidine base, or modified purine or pyrimidine bases. These terms include double-and single-stranded DNA, as well as double- and single-stranded RNA. Oligonucleotides of the invention may be used as primers and/or probes.

A nucleic acid, polynucleotide or oligonucleotide can comprise phosphodiester linkages or modified linkages including, but not limited to phosphotriester, phosphoramidate, siloxane, carbonate, carboxymethylester, acetamidate, carbamate, thioether, bridged phosphoramidate, bridged methylene-phosphonate, phosphorothioate, methylphosphonate, phosphorodithioate, bridged phosphorothioate or sulfone linkages, and combinations of such linkages.

A nucleic acid, polynucleotide or oligonucleotide can comprise the five biologically occurring bases (adenine, guanine, thymine, cytosine and uracil) and/or bases other than the five biologically occurring bases. These bases may serve a number of purposes, e.g., to stabilize or destabilize hybridization; to promote or inhibit probe degradation; or as attachment points for detectable moieties or quencher moieties. For example, a polynucleotide of the invention can contain one or more modified, non-standard, or derivatized base moieties, including, but not limited to, N6-methyl-adenine, N6-tert-butyl-benzyl-adenine, imidazole, substituted imidazoles, 5-fluorouracil, 5 bromouracil, 5-chlorouracil, 5-iodouracil, hypoxanthine, xanthine, 4-acetylcytosine, 5 (carboxyhydroxymethyl)uracil, 5 carboxymethylaminomethyl-2-thiouridine, 5 carboxymethylaminomethyluracil, dihydrouracil, beta-D-galactosylqueosine, inosine, N6 isopentenyladenine, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracil, 5-methoxyaminomethyl-2-thiouracil, beta-D mannosylqueosine, 5'-methoxycarboxymethyluracil, 5-methoxyuracil, 2-methylthio-N6- isopentenyladenine, uracil-5-oxyacetic acid (v), wybutoxosine, pseudouracil, queosine, 2 thiocytosine, 5-methyl-2-thiouracil, 2-thiouracil, 4-thiouracil, 5-methyluracil, uracil-5- oxyacetic acidmethylester, 3-(3-amino-3-N-2-carboxypropyl) uracil, (acp3)w, 2,6- diaminopurine, and 5-propynyl pyrimidine. Other examples of modified, non-standard, or derivatized base moieties may be found in U.S. Patent Nos. 6,001,611; 5,955,589; 5,844,106; 5,789,562; 5,750,343; 5,728,525; and 5,679,785.

Furthermore, a nucleic acid, polynucleotide or oligonucleotide can comprise one or more modified sugar moieties including, but not limited to, arabinose, 2-fluoroarabinose, xylulose, and a hexose.

### Introduction

The invention provides a V600E diagnostic assay for use in selecting cancer patients, *e.g*., melanoma cancer patients, colon cancer patients, thyroid cancer patients, and patients having low-grade serous ovarian cancers, who are candidates for treatment with a B-Raf inhibitor, such as a selective mutant B-Raf inhibitor, *e.g*., PLX4032. Thus, the diagnostic test can be used to classify patients according to their probability of responding to treatment with PLX4032.

Typically, the V600E mutation (also known as V599E (1796T>A)) is detected using a method that comprises determining the presence of a single-base mutation (T>A) at nucleotide position 1799 in codon 600 of exon 15. This mutation can also result from the two-base mutation TG>AA at nucleotide positions 1799-1800. The two-base mutation can also be detected by evaluating position 1799. In some embodiments, a nucleic acid may also be evaluated for the presence of a substitution at position 1800.

Other mutations also can occur at codon 600. These include V600K, V600D, and V600R. In some embodiments, a probe that detects a V600E mutation can also detect other codon 600 mutations, *e.g*., V600D, V600K and/or V600R. In some embodiments, a probe may also detect a mutation at codon 601.

The presence of a V600E mutation is typically determined by assessing nucleic acid, *e.g*., genomic DNA or mRNA, for the presence of a base substitution at position 1799. A wide variety of assays are available. In some embodiments, the assay is a 5" nuclease assay.

V600E can also be detected by detecting the presence of a mutant V600E B-Raf, *e.g*., using an immunoassay.

The presence of V600E indicates that the patient is a candidate for treatment of a B-Raf inhibitor, such as a mutant-specific B-Raf inhibitor. Therefore, the invention also comprises a method wherein a patient that is determined to have a V600E mutation is treated with a B-Raf inhibitor, such as a mutant-specific B-Raf inhibitor, *e.g*., PLX4032.

### Biological sample

The V600E mutation can be detected in various kinds of cancer, including melanoma; colorectal cancer; thyroid cancer, *e.g*., papillary thyroid cancer; and ovarian cancer, *e.g*., low-grade serous carcinoma. In some embodiments, V600E mutations are detected in lung cancer, gliomas, prostate cancer, breast cancer, sarcoma, liver cancer, pituitary cancer, and cancers that occur in the large intestine, biliary tract, eye, pancreas, stomach, central nervous system, hematopoetic and lymphoid tissue.

A V600E mutation is detected in a biological sample from a patient. The biological sample typically comprises a cancer cell. For example, the sample can be a tumor biopsy, *e.g*., of a malignant melanocytic neoplasm, a colorectal tumor, or a thyroid tumor; or from a tissue sample from a metastatic site. In other embodiments, the biological sample can be blood or another fluid, where the fluid comprises a cancer cell. In other embodiments, the biological sample can comprise circulating (cell-free) nucleic acids.

The mutation is often detected in nucleic acids that are obtained from the biological sample. The nucleic acid that is evaluated for the presence of a mutation can be RNA or DNA. In some embodiments, the mutation is detected in genomic DNA.

A biological sample can be obtained using any of a number of methods in the art. Further, a biological sample can be treated with a fixative such as formaldehyde and embedded in paraffin and sectioned for use. Alternatively, fresh or frozen tissue can be employed. In other embodiments, fine-needle aspirates may be used.

### Detection of a V600E mutation in a nucleic acid sequence

Detection techniques for evaluating nucleic acids for the presence of a V600E mutation involve procedures well known in the field of molecular genetics. Further, many of the methods involve amplification of nucleic acids. Ample guidance for performing such procedures is provided in the art. Exemplary references include manuals such as PCR Technology: Principles and Applications for DNA Amplification (ed. H. A. Erlich, Freeman Press, NY, N.Y., 1992); PCR Protocols: A Guide to Methods and Applications (eds. Innis, et al., Academic Press, San Diego, Calif., 1990); Current Protocols in Molecular Biology, Ausubel, 1994-1999, including supplemental updates through April 2004; Sambrook & Russell, Molecular Cloning, A Laboratory Manual (3rd Ed, 2001).

Although the methods typically employ PCR steps, other amplification protocols may also be used. Suitable amplification methods include ligase chain reaction *(see, e.g.,* Wu & Wallace, Genomics 4:560-569, 1988); strand displacement assay *(see, e.g.,* Walker et al., Proc. Natl. Acad. Sci. USA 89:392-396, 1992; U.S. Pat. No. 5,455,166); and several transcription-based amplification systems, including the methods described in U.S. Pat. Nos. 5,437,990; 5,409,818; and 5,399,491; the transcription amplification system (TAS) (Kwoh et al., Proc. Natl. Acad. Sci. USA 86:1173-1177, 1989); and self-sustained sequence replication (3SR) (Guatelli et al., Proc. Natl. Acad. Sci. USA 87:1874-1878, 1990; WO 92/08800). Alternatively, methods that amplify the probe to detectable levels can be used, such as Qβ-replicase amplification (framer & Lizardi, Nature 339:401-402, 1989; Lomeli et al., Clin. Chem. 35:1826-1831, 1989). A review of known amplification methods is provided, for example, by Abramson and Myers in Current Opinion in Biotechnology 4:41-47, 1993.

Typically, the detection of V600E is performed by assessing nucleic acids from the patient in an assay comprising oligonucleotide primers and/or probes. Oligonucleotides can be prepared by any suitable method, usually chemical synthesis. Oligonucleotides can be synthesized using commercially available reagents and instruments. Alternatively, they can be purchased through commercial sources. Methods of synthesizing oligonucleotides are well known in the art (*see, e.g,* Narang et al., Meth. Enzymol. 68:90-99, 1979; Brown et al., Meth. Enzymol. 68:109-151, 1979; Beaucage et al., Tetrahedron Lett. 22:1859-1862; 1981; and the solid support method of U.S. Pat. No. 4,458,066). In addition, modifications to the above-described methods of synthesis may be used to desirably impact enzyme behavior with respect to the synthesized oligonucleotides. For example, incorporation of modified phosphodiester linkages (e.g., phosphorothioate, methylphosphonates, phosphoamidate, or boranophosphate) or linkages other than a phosphorous acid derivative into an oligonucleotide may be used to prevent cleavage at a selected site. In addition, the use of 2'-amino modified sugars tends to favor displacement over digestion of the oligonucleotide when hybridized to a nucleic acid that is also the template for synthesis of a new nucleic acid strand.

Most assays for detecting a V600E mutation on the nucleic acid level entail one of several general protocols: hybridization using allele-specific oligonucleotides, primer extension, allele-specific ligation, sequencing, or electrophoretic separation techniques, *e.g*., singled-stranded conformational polymorphism (SSCP) and heteroduplex analysis. Exemplary assays include 5' nuclease assays, template-directed dye-terminator incorporation, molecular beacon allele-specific oligonucleotide assays, single-base extension assays, and mutations analysis using real-time pyrophosphate sequencing. Analysis of amplified sequences can be performed using various technologies such as microchips, fluorescence polarization assays, and matrix-assisted laser desorption ionization (MALDI) mass spectrometry. Two additional methods that can be used are assays based on invasive cleavage with Flap nucleases and methodologies employing padlock probes.

Determination of the presence or absence of a V600E allele is generally performed by analyzing a nucleic acid sample that is obtained from a biological sample comprising cancer cells from a patient to be analyzed. Often, the nucleic acid sample comprises genomic DNA. The genomic DNA is typically obtained from tumor samples, but may also be obtained from other cells or tissues, *e.g*., from metastatic site or blood.

The nucleic acid sample to be analyzed can also be RNA. For example, mRNA from a biological sample can be analyzed to determine the presence or absence of a V600E mutation. The nucleic acid sample is obtained from cells in which the target nucleic acid is expressed, *e.g*., a primary tumor or tissue from a metastatic site. Such an analysis can be performed by first reverse-transcribing the target RNA using, for example, a viral reverse transcriptase, and then amplifying the resulting cDNA; or using a combined high-temperature reverse-transcription-polymerase chain reaction (RT-PCR), as described in U.S. Pat. Nos. 5,310,652; 5,322,770; 5,561,058; 5,641,864; and 5,693,517.

Frequently used methodologies for analysis of nucleic acid samples to detect nucleotide substitutions are briefly described. However, any method known in the art can be used in the invention to detect the presence of nucleotide substitutions.

### Allele Specific Probes

Allele-specific hybridization relies on distinguishing between two DNA molecules differing by at least one base by hybridizing an oligonucleotide that is specific for one of the variant sequences to an amplified product obtained from amplifying the nucleic acid sample. An allele-specific assay may also comprise two allele-specific oligonucleotides, *e.g*., an allele-specific probe for the first variant and an allele-specific probe to the second variant where the probes differentially hybridize to one variant versus the other. Allele-specific hybridization typically employs short oligonucleotides, *e.g*., 15-35 nucleotides in length. Principles and guidance for designing such probe is available in the art, *e.g*., in the references cited herein. Hybridization conditions should be sufficiently stringent that there is a significant difference in hybridization intensity between alleles, and preferably an essentially binary response, whereby a probe hybridizes to only one of the alleles. Some probes are designed to hybridize to a segment of target DNA such that the site of interest, which is nucleotide position 1799 in codon 600 of exon 15 of *BRAF,* aligns with a central position (*e.g*., in a 15-base oligonucleotide at the 7 position; in a 16-based oligonucleotide at either the 8 or 9 position) of the probe, but this design is not required.

The amount and/or presence of an allele is determined by measuring the amount of allele-specific probe that is hybridized to the sample. Typically, the oligonucleotide is labeled with a label such as a fluorescent label. For example, an allele-specific probe that is specific for a V600E nucleotide substitution is hybridized to nucleic acids obtained from a biological sample under hybridization conditions that result in preferential hybridization to an allele. Fluorescence intensity is measured to determine if specific oligonucleotide has hybridized.

In one embodiment of the disclosure, the nucleotide present at the V600E position is identified by hybridization under sequence-specific hybridization conditions with an oligonucleotide probe exactly complementary to the mutant (or wild type) sequence of *BRAF* that comprises the V600E mutant site. The probe hybridizing sequence and sequence-specific hybridization conditions are selected such that a single mismatch at the mutation site destabilizes the hybridization duplex sufficiently so that it is effectively not formed. Thus, under sequence-specific hybridization conditions, stable duplexes will form only between the probe and the exactly complementary allelic sequence. Thus, oligonucleotides from about 10 to about 35 nucleotides in length, preferably from about 15 to about 35 nucleotides in length, which are exactly complementary to an allele sequence in a region which encompasses the mutation site are within the scope of the invention.

In an alternative embodiment of the disclosure, the nucleotide present at the mutation site is identified by hybridization under sufficiently stringent hybridization conditions with an oligonucleotide substantially complementary to the mutant or wild type allele in a region encompassing the mutation site, and exactly complementary to the allele at the mutation site. Because mismatches which occur at the sites that are not mutated, there are mismatches with both allele sequences, the difference in the number of mismatches in a duplex formed with the target allele sequence and in a duplex formed with the corresponding non-target allele sequence is the same as when an oligonucleotide exactly complementary to the target allele sequence is used. In this embodiment, the hybridization conditions are relaxed sufficiently to allow the formation of stable duplexes with the target sequence, while maintaining sufficient stringency to preclude the formation of stable duplexes with non-target sequences. Under such sufficiently stringent hybridization conditions, stable duplexes will form only between the probe and the target allele. Thus, oligonucleotides from about 10 to about 35 nucleotides in length, preferably from about 15 to about 35 nucleotides in length, which are substantially complementary to an allele sequence in a region which encompasses the mutation site, and are exactly complementary to the allele sequence at the mutation site, are within the scope of the invention.

The use of substantially, rather than exactly, complementary oligonucleotides may be desirable in assay formats in which optimization of hybridization conditions is limited. For example, in a multi-target immobilized-probe assay format, probes for each target are immobilized on a single solid support. Hybridizations are carried out simultaneously by contacting the solid support with a solution containing target DNA. As all hybridizations are carried out under identical conditions, the hybridization conditions cannot be separately optimized for each probe. The incorporation of mismatches into a probe can be used to adjust duplex stability when the assay format precludes adjusting the hybridization conditions. The effect of a particular introduced mismatch on duplex stability is well known, and the duplex stability can be routinely both estimated and empirically determined, as described above. Suitable hybridization conditions, which depend on the exact size and sequence of the probe, can be selected empirically using the guidance provided herein and well known in the art. The use of oligonucleotide probes to detect single base pair differences in sequence is described in, for example, Conner et al., 1983, Proc. Natl. Acad. Sci. USA 80:278-282, and U.S. Pat. Nos. 5,468,613 and 5,604,099.

The proportional change in stability between a perfectly matched and a single-base mismatched hybridization duplex depends on the length of the hybridized oligonucleotides. Duplexes formed with shorter probe sequences are destabilized proportionally more by the presence of a mismatch. In practice, oligonucleotides between about 15 and about 35 nucleotides in length are preferred for sequence-specific detection. Furthermore, because the ends of a hybridized oligonucleotide undergo continuous random dissociation and re-annealing due to thermal energy, a mismatch at either end destabilizes the hybridization duplex less than a mismatch occurring internally. Preferably, for discrimination of a single base pair change in target sequence, the probe sequence is selected which hybridizes to the target sequence such that the mutation site occurs in the interior region of the probe.

The above criteria for selecting a probe sequence that hybridizes to *BRAF* apply to the hybridizing region of the probe, *i.e*., that part of the probe which is involved in hybridization with the target sequence. A probe may be bound to an additional nucleic acid sequence, such as a poly-T tail used to immobilize the probe, without significantly altering the hybridization characteristics of the probe. One of skill in the art will recognize that for use in the present methods, a probe bound to an additional nucleic acid sequence which is not complementary to the target sequence and, thus, is not involved in the hybridization, is essentially equivalent to the unbound probe.

### 5'-nuclease assay

In some embodiments, the nucleic acid samples are assessed for the presence of a V600E mutation using a "TaqMan^{®}" or "5'-nuclease assay", as described in U.S. Pat. Nos. 5,210,015; 5,487,972; and 5,804,375; and Holland et al., 1988, Proc. Natl. Acad. Sci. USA 88:7276-7280. In the TaqMan^{®} assay, labeled detection probes that hybridize within the amplified region are present during the amplification reaction. The probes are modified so as to prevent the probes from acting as primers for DNA synthesis. The amplification is performed using a DNA polymerase having 5' to 3' exonuclease activity. During each synthesis step of the amplification, any probe which hybridizes to the target nucleic acid downstream from the primer being extended is degraded by the 5' to 3' exonuclease activity of the DNA polymerase. Thus, the synthesis of a new target strand also results in the degradation of a probe, and the accumulation of degradation product provides a measure of the synthesis of target sequences.

The hybridization probe employed in the assay can be an allele-specific probe that discriminates between the mutant and wild type alleles of *BRAF* at the V600E mutation site. Alternatively, the method can be performed using an allele-specific primer and a labeled probe that binds to amplified product.

Any method suitable for detecting degradation product can be used in a 5' nuclease assay. Often, the detection probe is labeled with two fluorescent dyes, one of which is capable of quenching the fluorescence of the other dye. The dyes are attached to the probe, preferably one attached to the 5' terminus and the other is attached to an internal site, such that quenching occurs when the probe is in an unhybridized state and such that cleavage of the probe by the 5' to 3' exonuclease activity of the DNA polymerase occurs in between the two dyes. Amplification results in cleavage of the probe between the dyes with a concomitant elimination of quenching and an increase in the fluorescence observable from the initially quenched dye. The accumulation of degradation product is monitored by measuring the increase in reaction fluorescence. U.S. Pat. Nos. 5,491,063 and 5,571,673 describe alternative methods for detecting the degradation of probe which occurs concomitant with amplification.

In one embodiment, a 5' nuclease assay to evaluate patient samples for the presence of the V 600E mutation in *BRAF* can be performed using the following primers, or sequences that are substantially identical to the primers:
TTS068-BRAF_F1: 5' CCTCACAGTAAAAATAGGTGATTTTGGTCTE 3' (E= t-butyl benzyl dA) (SEQ ID NO:25)
RL_BRAF_R5: 5'TAGCCTCAATTCTTACCATCCACAAAA 3' (SEQ ID NO:4).

Sequences that are substantially identical to the primer sequences include those that hybridize to the same complementary sequence. Thus, in some embodiments, primer sequences for use in the invention comprise at least 15 contiguous nucleotides, sometimes at least 16, 17, 18, 19, 20, 21, 22, 23, 24, 25, or 26 contiguous nucleotides of TTS068-BRAF_F1 or RL_BRAF_R5. In some embodiments, a primer has at least 27, 28, 29, or 30 contiguous nucleotide of TTS068-BRAF_F1. In other embodiments, primers for use in the invention have at least 80% identity, in some embodiments at least 85% identity, and in other embodiments at least 90% or greater identity to TTS068-BRAF_F1 or RL_BRAF_R5. In some embodiments, the forward primer is TTS068-BRAF-F1 and the reverse primer is a primer that allows for discrimination of the X chromosome pseudo gene, but does not overlap with RL_BRAF_R5, or has less than a 10 base pair over lap with RL_BRAF_R5. In some embodiments, the reverse primer can bind to a site that includes 20 nucleotides or less downstream of the binding site for RL_BRAF_R5. For example, reverse primers of the following sequences may also be employed:
5' A AAT AGC CTC AAT TCT TAC CAT CCA CAA AA 3' (SEQ ID NO:12)
5' TAG CCT CAA TTC TTA CCA TCC ACA AAA 3' (SEQ ID NO:13)
5' TAG CCT CAA TTC TTA CCA TCC ACA AAE 3' (SEQ ID NO: 1)
5' AGG GCC AAA AAT TTA ATC AGT GGA AAA A 3' (SEQ ID NO:15)
5' CAG TGG AAA AAT AGC CTC AAT TCT TAC CA 3' (SEQ ID NO:16)

In some embodiments, the forward primer can bind to a site that includes 20 bases upstream of the binding site of BRAF-F1. In some embodiments, the forward primer can be:
5' TTTCTTCATGAAGACCTCACAGTAAAAATE 3' (SEQ ID NO:17); or
5' ATATATTTCTTCATGAAGACCTCACAGTAAE 3' (SEQ ID NO:18).

A V600E mutation can also be detected where RNA is the starting template. Such an assay can comprise a reverse primer, *e.g*., a primer comprising a sequence:
5' ATG ACT TCT GGT GCC ATC CAC AA 3' (SEQ ID NO:19).

Other reverse primers that can be employed include:
5' AAA AAT AGC CTC AAT TCT TAC CAT CCA CAA AA 3' (SEQ ID NO:20);
5' GCC ATC CAC AAA ATG GAT CCA GAC A3' (SEQ ID NO:21); or
5' CAA AAT GGA TCC AGA CAA CTG TTC AAA 3' (SEQ ID NO:22).

In one embodiment of the invention, a 5' nuclease assay is performed using one or both of the following allele-specific probes, which detect either a mutant (TTS 155-BRAF_MU) or wild type (TTS148-BRAF_WTs) sequence:
TTS155-BRAF_MU 5' QCTACAIAIFAAATCTCGATGGAGTGGGTCCCAP 3' (SEQ ID NO:23)
TTS 148-BRAF_WT 5' QACAITGEAAATCTCGATGGAGTGGGTCCCAP 3' (SEQ ID NO:24)
(E = HEX Reporter Dye, F= FAM Reporter Dye, I = deoxyinosine, Q = BHQ2 Quencher Dye, P= 3'-Phosphate). The dye (F or E) is inserted between either dI and dA (TTS155-BRAF_MU) or between dG and dA (TTS148-BRAF_WT).

As understood in the art, a TTS155-BRAF_MU or TTS148-BRAF_WT probe may also include modifications, *e.g*., the particular fluorescent dye, the quencher, and/or the positions of the dye, that are different from those depicted above.

In some embodiments, the probe that detects V600E, *e.g*., TTS155-BRAF_MU, also detects V600D (1799_1800TG>AT) and V600K (1798_1799GT>AA). In some embodiments, a probe that detects a V600E mutation also detects K601 E (1801A>G) and V600R (1798_1799GT>AG).

In some embodiments of the disclosure, a sequence substantially identical to a probe sequence can be used. Sequences that are substantially identical to the probe sequences include those that hybridize to the same complementary sequence as the probe. Thus, in some embodiments, probe sequences for use in the invention comprise at least 15 contiguous nucleotides, sometimes at least 16, 17, 18, 19, 20, 21, 22, 23, 24, 25, 26, 27, 28, 29, or 30 contiguous nucleotides of the TTS 155-BRAF_MU or TTS 148-BRAF_WT. In some embodiments, a primer has at least 27, 28, 29, or 30 contiguous nucleotide of TTS 155-BRAF_MU or TTS148-BRAF_WT. In other embodiments, primers for use in the invention have at least 80% identity, in some embodiments at least 85% identity, and in other embodiments at least 90% or greater identity to TTS155-BRAF_MU or TTS 148-BRAF_WT.

A 5' nuclease assay for the detection of a V600E mutation in *BRAF* can employ any polymerase that has a 5' to 3' exonuclease activity. Thus, in some embodiments, the polymerases with 5'-nuclease activity are thermostable and thermoactive nucleic acid polymerases. Such thermostable polymerases include, but are not limited to, native and recombinant forms of polymerases from a variety of species of the eubacterial genera Thermus, Thermatoga, and Thermosipho, as well as chimeric forms thereof. For example, Thermus species polymerases that can be used in the methods of the invention include *Thermus aquaticus* (Taq) DNA polymerase, *Thermus thermophilus* (Tth) DNA polymerase, Thermus species Z05 (Z05) DNA polymerase, Thermus species sps17 (sps17), and *Thermus* species Z05 (*e.g*., described in U.S. Pat. Nos. 5,405,774; 5,352,600; 5,079,352; 4,889,818; 5,466,591; 5,618,711; 5,674,738, and 5,795,762). Thermatoga polymerases that can be used in the methods of the invention include, for example, *Thermatoga maritima* DNA polymerase and *Thermatoga neapolitana* DNA polymerase, while an example of a Thermosipho polymerase that can be used is *Thermosipho africanus* DNA polymerase. The sequences of Thermatoga maritima and Thermosipho africanus DNA polymerases are published in International Patent Publication WO 92/06200. The sequence of Thermatoga neapolitana may be found in International Patent Publication No. WO 97/09451.

In the 5' nuclease assay, the amplification detection is typically concurrent with amplification (*i.e*., "real-time"). In some embodiments the amplification detection is quantitative, and the amplification detection is real-time. In some embodiments, the amplification detection is qualitative (*e.g*., end-point detection of the presence or absence of a target nucleic acid). In some embodiments, the amplification detection is subsequent to amplification. In some embodiments, the amplification detection is qualitative, and the amplification detection is subsequent to amplification.

The probe can be labeled with any number of labels, but is typically a fluorescent label. In some embodiments, the fluorophore moiety is selected from the group consisting of fluorescein-family dyes, polyhalofluorescein-family dyes, hexachlorofluorescein-family dyes, coumarin-family dyes, rhodamine-family dyes, cyanine-family dyes, oxazine-family dyes, thiazine-family dyes, squaraine-family dyes, chelated lanthanide-family dyes, azo-family dyes, triphenylmethane-family dyes, and BODIPY®-family dyes.

The assay often comprises a probe labeled with a fluorescent label and a quencher moiety. In some embodiments, the quencher moiety is selected from the group consisting of fluorescein-family dyes, polyhalofluorescein-family dyes, hexachlorofluorescein-family dyes, coumarin-family dyes, rhodamine-family dyes, cyanine-family dyes, oxazine-family dyes, thiazine-family dyes, squaraine-family dyes, chelated lanthanide-family dyes, BODIPY®-family dyes, azo-family dyes, triphenylmethane-family dyes, low-fluorescent quencher moieties (*i.e*., "dim donors") and non-fluorescent quencher moieties (*e.g*., so-called "dark quenchers" including Black Hole Quenchers™ (BHQ)).

In one embodiment, the fluorophore moiety is a fluorescein-family dye and the quencher moiety is a cyanine-family dye. In one embodiment, the fluorophore moiety is a fluorescein-family dye and the quencher moiety is a hexachlorofluorescein-family dye. In one embodiment, the fluorophore moiety is a hexachlorofluorescein-family dye and the quencher moiety is a cyanine-family dye. In one embodiment, the quencher is a dark quencher, for example, a BHQ-1, a BHQ-2 or a BHQ-3 (Biosearch Technologies, Novato, CA). In one embodiment, the fluorophore moiety is a fluorescein-family dye or a cyanine-family dye and the quencher moiety is a dark quencher.

### Immobilized supports

In some embodiments, allele-specific hybridization is performed in an assay format using an immobilized target or immobilized probe. Such formats are known in the art and include, *e.g*., dot-blot formats and reverse dot blot assay formats that are described in U.S. Pat. Nos. 5,310,893; 5,451,512; 5,468,613; and 5,604,099.

### Allele-Specific Primers

The presence or absence of a V600E mutation can be detected using allele-specific amplification or primer extension methods. These reactions typically involve use of primers that are designed to specifically target the mutant (or wild type) site via a mismatch at the 3' end of a primer, *e.g*., at the 3' nucleotide or penultimate 3' nucleotide. The presence of a mismatch effects the ability of a polymerase to extend a primer when the polymerase lacks error-correcting activity. For example, to detect a V600E mutant sequence using an allele-specific amplification- or extension-based method, a primer complementary to the mutant A allele at nucleotide position 1799 in codon 600 of *BRAF* is designed such that the 3' terminal nucleotide hybridizes at the mutant position. The presence of the mutant allele can be determined by the ability of the primer to initiate extension. If the 3' terminus is mismatched, the extension is impeded. Thus, for example, if a primer matches the mutant allele nucleotide at the 3' end, the primer will be efficiently extended. Amplification may also be performed using an allele-specific primer that is specific from the *BRAF* wild type sequence at position 1799.

Typically, the primer is used in conjunction with a second primer in an amplification reaction. The second primer hybridizes at a site unrelated to the mutant position. Amplification proceeds from the two primers leading to a detectable product signifying the particular allelic form is present. Allele-specific amplification- or extension-based methods are described in, for example, WO 93/22456; U.S. Pat. Nos. 5,137,806; 5,595,890; 5,639,611; and U.S. Pat. No. 4,851,331.

Using allele-specific amplification-based genotyping, identification of the alleles requires only detection of the presence or absence of amplified target sequences. Methods for the detection of amplified target sequences are well known in the art. For example, gel electrophoresis and probe hybridization assays described are often used to detect the presence of nucleic acids.

In an alternative probe-less method, the amplified nucleic acid is detected by monitoring the increase in the total amount of double-stranded DNA in the reaction mixture, is described, *e.g*., in U.S. Pat. No. 5,994,056; and European Patent Publication Nos. 487,218 and 512,334. The detection of double-stranded target DNA relies on the increased fluorescence various DNA-binding dyes, *e.g*., SYBR Green, exhibit when bound to double-stranded DNA.

As appreciated by one in the art, allele-specific amplification methods can be performed in reaction that employ multiple allele-specific primers to target particular alleles. Primers for such multiplex applications are generally labeled with distinguishable labels or are selected such that the amplification products produced from the alleles are distinguishable by size. Thus, for example, both wild type and mutant V600E alleles in a single sample can be identified using a single amplification reaction by gel analysis of the amplification product.

An allele-specific oligonucleotide primer may be exactly complementary to one of the alleles in the hybridizing region or may have some mismatches at positions other than the 3' terminus of the oligonucleotide. For example the penultimate 3' nucleotide may be mismatched in an allele-specific oligonucleotide. In other embodiments, mismatches may occur at (non-mutant) sites in both allele sequences.

### Additional V600E BRAF nucleic acid mutation detection methods

The presence (or absence) of a V600E mutation can also be detected by direct sequencing. Methods include dideoxy sequencing-based methods and methods such as Pyrosequencing™ of oligonucleotide-length products. Such methods often employ amplification techniques such as PCR. Another similar method for sequencing does not require use of a complete PCR, but typically uses only the extension of a primer by a single, fluorescence-labeled dideoxyribonucleic acid molecule (ddNTP) that is complementary to the nucleotide to be investigated. The nucleotide at the polymorphic site can be identified via detection of a primer that has been extended by one base and is fluorescently labeled (*e.g*., Kobayashi et al, Mol. Cell. Probes, 9:175-182, 1995).

Amplification products generated using an amplification reaction can also be analyzed by the use of denaturing gradient gel electrophoresis: Different alleles can be identified based on the different sequence-dependent melting properties and electrophoretic migration of DNA in solution (*see*, *e.g.,* Erlich, ed., PCR Technology, Principles and Applications for DNA Amplification, W. H. Freeman and Co, New York, 1992, Chapter 7).

In other embodiments, alleles of target sequences can be differentiated using single-strand conformation polymorphism analysis, which identifies base differences by alteration in electrophoretic migration of single stranded PCR products, as described, *e.g,* in Orita et al., Proc. Nat. Acad. Sci. 86, 2766-2770 (1989). Amplified PCR products can be generated as described above, and heated or otherwise denatured, to form single stranded amplification products. Single-stranded nucleic acids may refold or form secondary structures which are partially dependent on the base sequence. The different electrophoretic mobilities of single-stranded amplification products can be related to sequence differences between alleles of target regions.

The methods used to detect the presence of a V600E mutation in *BRAF*, typically employ labeled oligonucleotides, *e.g*., fluorescent labels, as described above. Oligonucleotides can be labeled by incorporating a label detectable by spectroscopic, photochemical, biochemical, immunochemical, or chemical means. Useful labels include fluorescent dyes, radioactive labels, *e.g*., ³²P, electron-dense reagents, enzyme, such as peroxidase or alkaline phosphatase, biotin, or haptens and proteins for which antisera or monoclonal antibodies are available. Labeling techniques are well known in the art (*see*, *e.g.,* Current Protocols in Molecular Biology, *supra*; Sambrook & Russell, *supra*).

### Detection of protein variants

A V600E mutation in B-Raf can also be detected by methods that discriminate between wild type and mutant B-Raf. Often these methods employ an antibody specific to mutant B-Raf.

A general overview of the applicable technology can be found in Harlow & Lane, Antibodies: A Laboratory Manual (1988) and Harlow & Lane, Using Antibodies (1999). Methods of producing polyclonal and monoclonal antibodies that react specifically with an allelic variant are known to those of skill in the art (*see, e.g*., Coligan, Current Protocols in Immunology (1991); Harlow & Lane, *supra*; Goding, Monoclonal Antibodies: Principles and Practice (2d ed. 1986); and Kohler & Milstein, Nature 256:495-497 (1975)). Such techniques include antibody preparation by selection of antibodies from libraries of recombinant antibodies in phage or similar vectors, as well as preparation of polyclonal and monoclonal antibodies by immunizing rabbits or mice (*see, e.g.,* Huse et al., Science 246:1275-1281 (1989); Ward et al., Nature 341:544-546 (1989)).

The mutant B-Raf can be detected by a variety of immunoassay methods. For a review of immunological and immunoassay procedures, see Basic and Clinical Immunology (Stites & Terr eds., 7th ed. 1991). Moreover, the immunoassays of the present invention can be performed in any of several configurations, which are reviewed extensively in Enzyme Immunoassay (Maggio, ed., 1980); and Harlow & Lane, *supra.* For a review of the general immunoassays, see also Methods in Cell Biology: Antibodies in Cell Biology, volume 37 (Asai, ed. 1993); Basic and Clinical Immunology (Stites & Terr, eds., 7th ed. 1991).

Commonly used assays include noncompetitive assays, *e.g*., sandwich assays, and competitive assays. Typically, an assay such as an ELISA assay can be used. The amount of the polypeptide variant can be determined by performing quantitative analyses.

Other detection techniques, *e.g*., MALDI, may be used to directly detect the presence of V600E in B-Raf.

A sample for analysis is obtained from cancer cells, *e.g*., tumor tissue, from the patient.

### Treatment

The mutation detection method disclosed herein can include the use of data analysis software that will inform the user whether the patient should be treated or not with a B-Raf inhibitor, such as a mutation-specific B-Raf inhibitor, *e.g*., PLX4032, based on the presence or absence, respectively, of the B-Raf V600E mutation.

A patient that is determined to be positive for the presence of a mutation at amino acid position 600, *e.g*., a V600E mutation, is a candidate for treatment with a B-Raf kinase inhibitor, *e.g*., a mutant specific B-Raf kinase inhibitors such as PLX4032. Various B-Raf kinase inhibitors, including mutant-specific B-Raf kinase inhibitors, are described, e.g., in WO 2007/002325 and WO 2007/002433. PLX4032 is referred to in WO 2007/002433 and WO 2007/002325 as "P-0956".

Direction for administration of B-Raf kinase inhibitors, *e.g*., a mutant-specific B-Raf kinase inhibitor, can be found, *e.g*., in WO/2007/002325, and WO/2007/002433. Suitable dosage forms, in part, depend upon the use or the route of administration, for example, oral, transdermal, transmucosal, inhalant or by injection (parenteral). Such dosage forms should allow the compound to reach target cells. Other factors are well known in the art, and include considerations such as toxicity and dosage forms that retard the compound or composition from exerting its effects. Techniques and formulations generally may be found in The Science and Practice of Pharmacy, 21st edition, Lippincott, Williams and Wilkins, Philadelphia, PA, 2005.

Pharmaceutical compositions can include carriers or excipients. The carriers or excipients can be chosen to facilitate administration of the compound. Examples of carriers include calcium carbonate, calcium phosphate, various sugars such as lactose, glucose, or sucrose, or types of starch, cellulose derivatives, gelatin, vegetable oils, polyethylene glycols and physiologically compatible solvents. Examples of physiologically compatible solvents include sterile solutions of water for injection (WFI), saline solution, and dextrose.

The compounds can be administered by different routes including intravenous, intraperitoneal, subcutaneous, intramuscular, oral, transmucosal, rectal, transdermal, or inhalant. In some embodiments, the specific B-Raf kinase inhibitor is administered orally. For oral administration, for example, the compounds can be formulated into conventional oral dosage forms such as capsules, tablets, and liquid preparations such as syrups, elixirs, and concentrated drops.

For inhalants, B-Raf inhibitors may be formulated as dry powder or a suitable solution, suspension, or aerosol. Powders and solutions may be formulated with suitable additives known in the art. For example, powders may include a suitable powder base such as lactose or starch, and solutions may comprise propylene glycol, sterile water, ethanol, sodium chloride and other additives, such as acid, alkali and buffer salts. Such solutions or suspensions may be administered by inhaling via spray, pump, atomizer, or nebulizer, and the like.

Alternatively, B-Raf inhibitors can be injected (parenteral administration) may be used, e.g., intramuscular, intravenous, intraperitoneal, and/or subcutaneous. For injection, the inhibitors are formulated in sterile liquid solutions, preferably in physiologically compatible buffers or solutions, such as saline solution, Hank's solution, or Ringer's solution. In addition, the compounds may be formulated in solid form and redissolved or suspended immediately prior to use. Lyophilized forms can also be produced.

Administration can also be by transmucosal, topical, or transdermal means. For transmucosal, topical or transdermal administration, penetrants appropriate to the barrier to be permeated are used in the formulation. Such penetrants are generally known in the art, and include, for example, for transmucosal administration, bile salts and fusidic acid derivatives. In addition, detergents may be used to facilitate permeation. Transmucosal administration, for example, may be through nasal sprays or suppositories (rectal or vaginal). The topical compositions of this invention are formulated preferably as oils, creams, lotions, ointments and the like by choice of appropriate carriers known in the art. Suitable carriers include vegetable or mineral oils, white petrolatum (white soft paraffin), branched chain fats or oils, animal fats and high molecular weight alcohol. The preferred carriers are those in which the active ingredient is soluble. Emusifiers, stabilizers, humectants and antioxidants may also be included as well as agents imparting color or fragrance, if desired. Creams for topical application can be formulated from a mixture of mineral oil, self-emulsifying beeswax and water in which mixture the active ingredient, dissolved in a small amount solvent (e.g., an oil), is admixed. Additionally, administration by transdermal means may comprise a transdermal patch or dressing such as a bandage impregnated with an active ingredient and optionally one or more carriers or diluents known in the art. To be administered in the form of a transdermal delivery system, the dosage administration will, of course, be continuous rather than intermittent throughout the dosage regimen.

The amounts of various compounds to be administered can be determined by standard procedures taking into account factors such as the compound IC_{5O}, the biological half-life of the compound, the age, size, and weight of the subject, and various other parameters associated with the subject. The importance of these and other factors are well known to those of ordinary skill in the art. Generally, a dose will be between about 0.01 and 50 mg/kg, preferably 0.1 and 20 mg/kg of the subject being treated. Multiple doses may be used.

B-Raf inhibitors can also be used in conjunction with other cancer therapies.

### Kits

The invention also provides kits comprising useful components for practicing the methods, comprising an allele-specific probe comprising SEQ ID NO:1 wherein "n" is deoxyinosine. In some embodiments, the kit may comprise one or more oligonucleotides probes that are specific for the mutant or V600E allele. Such a kit can also contain amplification primers for amplifying a region of *BRAF* locus that encompasses the V600E mutation site. Thus, in some embodiments, a kit comprises the primer set
TTS068-BRAF_F1: 5' CCTCACAGTAAAAATAGGTGATTTTGGTCTE 3' (E= t-butyl benzyl dA) (SEQ ID NO:25) and
RL_BRAF_R5: 5' TAGCCTCAATTCTTACCATCCACAAAA 3' (SEQ ID NO:4),
which amplify a target region of *BRAF.* The kit can further comprises probes, such as the allele-specific probes. Examples of allele-specific probes that can be used in a kit of the invention are:
TTS155-BRAF_MU 5' QCTACAIAIFAAATCTCGATGGAGTGGGTCCCAP 3' (SEQ ID NO:23)
TTS 148-BRAF_WT 5' QACAITGEAAATCTCGATGGAGTGGGTCCCAP 3' (SEQ ID NO:24).

A kit can also comprise primers and/or probes that are substantially identical to the noted oligonucleotides. In some embodiments, a kit for use in the invention comprises one or more oligonucleotides that comprise at least 15 contiguous nucleotides of the nucleic acid sequence of, SEQ ID NO:2, SEQ ID NO:3, and/or SEQ ID NO:4.

Other optional components of the kits include additional reagents for determining the presence of nucleotide substitutions. For example, a kit can contain a polymerase, substrate nucleoside triphosphates and appropriate buffers for amplification reactions, and instructions for carrying out the present method. Other kit components can include DNA extraction reagents and protocol and divalent ion cofactor. The buffer can include, *e.g*., UNG. In some embodiments, the buffer can include aptamer.

A kit may also include controls, such as V600E DNA controls.

### EXAMPLES

### Detection of B-Raf V600E mutation

This example demonstrates a real-time PCR (polymerase chain reaction)-based diagnostic test for the measurement of the B-Raf V600E (BRAF nucleotide 1799 T >A) mutation in genomic DNA extracted from formalin-fixed paraffin embedded (FFPE) cancer tumor tissues. In this example, genomic DNA extracted from FFPE tissue was tested by TaqMan PCR analysis. For the PCR reaction, a Master Mix was made by combining TaqMan RNA Reaction Mix, Primer-Probe Mix and magnesium acetate (Mg(OAc)₂) cofactor. Amplification was performed using 125 ng of genomic DNA. Amplification products were detected using a COBAS TaqMan 48 Analyzer.

The genomic region containing the B-Raf V600E site (BRAF 1799 T>A) in exon 15 was amplified, producing a 116-base pair double-stranded DNA (amplicon). The amplification reaction was performed for 55 cycles. The amplification and detection system measured, in real-time, the amounts of PCR products generated at each cycle of amplification by measuring the fluorescent signal resulting from cleavage of the target-specific probes. The target-specific B-Raf wild-type (WT; V600) and B-Raf V600E mutation probes were each labeled with a different reporter dye. Wavelength-specific measurement of the fluorescence emitted by these reporter dyes during each amplification cycle permitted identification of B-Raf WT and V600E amplicons in a single reaction tube. Once fluorescent signal from each reporter dye reached a predefined threshold level, cycle-to-threshold (Ct) values were calculated for both the WT and V600E mutation allele in the multiplex reaction.

*BRAF* primers were designed to amplify a *BRAF* exon 15 region of 115 base pairs (bp). Using the UCSC Genome Browser's BLAT tool (http://www.genome.ucsc.edu/cgi-bin/hgBlat?command=start) on the Human Genome March 2006 Assembly, *BRAF* exon 15 has 93.4% homology with a chromosome X (chrX) sequence chrX:74721094-74721213, an apparent pseudo gene. A reverse primer that includes a portion of *BRAF* intron 15 was used to specifically amplify the intended *BRAF* sequence. As shown in Figure 1, the reverse primer has only 55.6% (15 of 27 nucleotides) homology with the chromosome X sequence. The probes in the *BRAF* Primer-Probe Mix are complementary to the amplicon strand resulting from extension of this reverse primer, further ensuring that the test result is specific to the *BRAF* sequence.

Primers were synthesized on an ABI 394 DNA Synthesizer (Applied Biosystems Inc., Foster City, CA) using standard deoxynucleotide phosphoramidites, dicyanoimidazole (DCI) as activator, and standard synthesis cycles (with minor modifications) in the conventional 3' to 5' orientation on a solid phase controlled pore glass (CPG) support. The 3'-t-Butyl Benzyl dA was introduced as a modified nucleoside, as part of the CPG ( Roche Applied Sciences, Penzberg, Germany) used as the solid support for the DNA synthesis. Following the addition of the last base, the 5'-dimethoxytrityl (DMT) protecting group was removed on the synthesizer and the oligonucleotide was deprotected with ammonium hydroxide at 55°C for 16 hours. The crude oligonucleotide was evaporated to remove the ammonia and purified using Mono-Q HR 16/10 strong anion exchange high pressure liquid chromatography (HPLC) column (Amersham Biosciences, Piscataway, NJ) with a linear gradient of sodium chloride, at high pH. Fractions were analyzed using a DNAPac PA100 (Dionex Inc., Sunnyvale, CA) ion exchange column and pooled using a minimum purity criterion of >90%. The pooled fractions were desalted and formulated in 10 mM Tris, pH 8.0. The following primers were synthesized for the PCR reaction:
TTS068-BRAF_F1: 5' CCTCACAGTAAAAATAGGTGATTTTGGTCTE 3' (E= t-butyl benzyl dA) (SEQ ID NO:25) and
RL_BRAF_R5: 5' TAGCCTCAATTCTTACCATCCACAAAA 3' (SEQ ID NO:4).

TaqMan probes were synthesized on an ABI 394 DNA Synthesizer (Applied Biosystems Inc.) using ultramild deoxynucleotide phosphoramidites (Pierce Biotechnology, Milwaukee, WI), DCI as activator, and standard synthesis cycles (with minor modifications) in the conventional 3' to 5' orientation on a solid phase CPG support. Fluorescent label, cx-FAM (6-carboxyfluorescein, Biogenex Inc.) or cx-HEX (6-carboxyhexachlorofluorescein (Biogenex Inc., San Ramon, CA), and Black Hole Quencher (BHQ-2) (Biosearch Inc., Novato, CA) quencher were incorporated using phosphoramidite reagents and 10-minute coupling cycles. The 3'-phosphate was introduced by means of 3'-extension Blocker CPG (Clontech Inc., Mountain View, CA). Following synthesis, the DMT protecting group was removed on the synthesizer and the oligonucleotide was deprotected with ammonium hydroxide at ambient temperature for 16 hours. The crude oligonucleotide was purified using Mono-Q HR 16/10 strong anion exchange HPLC column (Amersham Biosciences) with a linear gradient of sodium chloride. Fractions were analyzed using a DNAPac PA100 (Dionex Inc., Sunnyvale, CA) ion exchange column and pooled using a minimum purity criterion of >90%. The pooled fractions were desalted and formulated in probe storage buffer comprising 80 mM Tricine, pH 8.2, 240 mM potassium acetate, 0.1 mM EDTA, and 0.09% sodium azide. The Taqman^{®} probes are:
V600E (TTS155-BRAF_MU): 5'
   QCTACAIAIFAAATCTCGATGGAGTGGGTCCCAP 3' (SEQ ID NO:23)
wild type (TTS148-BRAF_WT): 5'
   QACAITGEAAATCTCGATGGAGTGGGTCCCAP 3' (SEQ ID NO:24)
(E = HEX reporter eye, F= FAM reporter eye, I = deoxyinosine, Q = BHQ2 quencher dye, P= 3'-phosphate).

The amplification reaction also included AmpErase (uracil-N-glycosylase, UNG) and deoxyuridine triphosphate (dUTP). AmpErase recognizes and catalyzes the destruction of DNA strands containing deoxyuridine, but not DNA containing thymidine. Deoxyuridine is not present in naturally occurring DNA, but is always present in amplicon due to the use of deoxyuridine triphosphate in place of thymidine triphosphate as one of the dNTPs in the PCR Reaction Mix reagent. The incorporation of UNG in the Reaction Mix minimizes contamination caused by amplicon carryover. AmpErase will not degrade target amplicon if and probes, and test kits.

### Detection of PCR Products

The BRAF primer-probe mix employed in this analysis contained two dual-labeled fluorescent probes that are specific for the nucleotide sequences encoding B-Raf WT or V600E, respectively, enabling real-time detection of specific PCR product accumulation through the release of fluorescent signal during the amplification process. The B-Raf WT and V600E probes are labeled with different fluorescent reporter dyes and the same quencher dye. When the probes are intact, the reporter dye fluorescence is suppressed by the quencher dye due to Förster-type energy transfer. During PCR, each probe hybridizes in a sequence-dependent manner to its target sequence and is cleaved by the 5'-nuclease activity of the Z05 DNA polymerase, separating the two dyes. Once the reporter and quencher dyes are separated, fluorescence from the reporter dye is detectable. With each PCR cycle generating more amplicon, the cumulative signal from the reporter dye is effectively increased. Amplification of the B-Raf WT and V600E sequences was monitored independently by measuring each probe at its corresponding specific emission wavelength range during each cycle.

Each genomic DNA sample was amplified in a 100 µL reaction comprising 50 µL of a working master mix, which was made by adding 17 µL of primer-probe mix and 13 µL of 25 mM Mg(OAc)₂ cofactor to 20 µL of TaqMan RNA reaction mix per sample. A genomic DNA sample (125ng) was added in a volume of 50 µL to the working master mix. Each sample was amplified in duplicate. The amplification reactions were performed under the conditions shown in Table 1. Each run included a positive control reaction for V600E and a wild type positive control reaction, as well as a negative control reaction.

**Table 1: Thermal Cycling Profile**

| **Step** | **Description** | **Temperature** | **Time** | **Number of Cycles** |
|---|---|---|---|---|
| 1 | UNG Decontamination | 50°C | 5 min. | 1X |
| 2 | Pre-Cycling Denaturation | 95°C | 1 min. | 1X |
| 3 | Denaturation 1 | 95°C | 15 sec. | 2X |
| | Annealing/Extension 1 | 61°C | 30sec. | |
| 4 | Denaturation 2 | 92°C | 15 sec. | 53X |
| | Annealing/Extension 2 | 61°C | 30 sec. | |
| 5 | Post-Cycling Hold | 40°C | 2 min. | 1X |

### Data analysis

Ct values for B-Raf V600E (channel 1) and WT (channel 2) were calculated by AmpliLink 3.1 software on the COBAS TaqMan 48 Analyzer workstation. The Ct values from the negative and positive control reactions were used to determine if a run was valid. Table 2 lists the acceptable Ct values for each control reaction.

**Table 2: Acceptable Ct Values for Control Reactions**

| **a.** | **b. Channel 1** | **d. Channel 2** |
|---|---|---|
| | **c. (FAM, V600E probe)*** | **e. (HEX, WT probe)*** |
| **f. Negative** | g. Ct = -1 | h. Ct = -1 |
| **i. Positive Control #1 (V600E Plasmid)** | j. Ct between 29 and 33 | k. C =-1 |
| **1. Positive Control #2 (WT Plasmid)** | m. Ct = -1 | n. Ct between 28 and 32 |

| | | |
|---|---|---|
| * A Ct value of -1 indicates that no significant amplification was detected. | | |

For valid runs, the Ct value for each sample was evaluated against acceptable ranges for each channel that have been defined by analytical performance studies. Table 3 provides an example of how each pair of Ct values was translated into a B-Raf V600E-mutation status call.

**Table 3: Table of Ct Values for Assigning V600E Status.**

| **o. Channel 1** | **q. conditional** | **r. Channel 2** | **t. V600E Status** |
|---|---|---|---|
| **p. (FAM, V600E probe)*** | | **s. (HEX, WT probe)*** | |
| u. Ct = -1 | v. and | w. Ct | x. Negative |
| y. 15 ≤ Ct ≤ 50 | z. and | aa. 15 ≤ Ct ≤ 40 | bb. Positive |
| cc. Ct <15 or >50 | dd. or | ee. Ct <15 or >40 | ff. Indeterminate |

| | | | |
|---|---|---|---|
| * A Ct value of -1 indicates that no significant amplification was detected. Key: E = HEX Reporter Dye, F= FAM Reporter Dye, I = deoxyinosine, Q = BHQ2 Quencher Dye, P= 3'-Phosphate | | | |

The following samples were evaluated. The TaqMan^{®} PCR results were validated by sequencing.

**Table 4. Clinical Sample Characteristics**

| **Sample ID** | **Diagnosis** | **% Tumor** |
|---|---|---|
| M1 | PRIMARY MELANOMA VERTICAL GROWTH PHASE | 60 |
| M2 | MELANOMA | 95 |
| M3 | PRIMARY MELANOMA VERTICAL GROWTH PHASE | 75 |
| M4 | MELANOMA, | 50 |
| M5 | PRIMARY MELANOMA VERTICAL GROWTH PHASE | 85 |
| M6 | PRIMARY MELANOMA VERTICAL GROWTH PHASE | 90 |
| M7 | MELANOMA | 90 |
| M8 | PRIMARY MELANOMA VERTICAL GROWTH PHASE | 90 |
| M9 | PRIMARY MELANOMA VERTICAL GROWTH PHASE | 90 |
| M10 | PRIMARY MELANOMA RADIAL GROWTH PHASE | 95 |
| RM1 | Metastatic melanoma (LN) | 10 |
| RM2 | Metastatic melanoma (LN) | 45 |
| RM3 | Metastatic melanoma (LN) | 30 |
| RM4 | Metastatic melanoma (LN) | 10 |
| RM5 | Metastatic melanoma (LN) | 50 |
| RM6 | Metastatic melanoma (LN) | 30 |
| RM7 | Metastatic melanoma (LN) | 50 |
| RM8 | Metastatic melanoma (LN) | 40 |
| RM9 | Metastatic melanoma (LN) | 40 |
| RM10 | Metastatic melanoma (LN)? | 10 |
| RM11 | Metastatic melanoma (LN) | 75 |
| RM12 | Metastatic melanoma (LN) | 55 |
| RM13 | Metastatic melanoma (LN) | 30 |
| RM14 | Metastatic melanoma | 50 |
| RM15 | Metastatic melanoma (LN) | 80 |
| RM16 | Metastatic melanoma (LN) | 45 |
| RM17 | Metastatic melanoma (LN) | 50 |
| RM18 | Metastatic melanoma (LN) | 50 |
| RM19 | Metastatic melanoma (LN) | 50 |
| RM20 | Metastatic melanoma (LN) | 35 |

DNA was extracted from thirty FFPE melanoma specimens (Table 4). The resulting samples contained a mixture of DNA from both tumor and normal cells. The mutation detection results from the TaqMan^{®} assay in comparison with pyrosequencing and GS20 sequencing are shown in Table 5.

**Table 5 V600E Status as Determined by Three Independent Methods**

| **Sample ID** | **TaqMan®** | **Pyrosequencing** | **GS20 Sequencing** |
|---|---|---|---|
| M1 | negative | negative | negative |
| M2 | negative | negative | negative |
| M3 | negative | failed | negative |
| M4 | positive | positive | positive |
| M5 | positive | positive | positive |
| M6 | positive | positive | positive |
| M7 | positive | positive | positive |
| M8 | negative | negative | negative |
| M9 | negative | negative | negative |
| M10 | negative | negative | negative |
| RM1 | negative | failed | negative |
| RM2 | negative | negative | negative |
| RM3 | positive | negative | positive |
| RM4 | negative | negative | negative |
| RM5 | positive | positive | positive |
| RM6 | positive | negative | positive |
| RM7 | negative | negative | negative |
| RM8 | positive | negative | positive |
| RM9 | negative | negative | negative |
| RM10 | negative | negative | negative |
| RM11 | negative | negative | negative |
| RM12 | negative | negative* | negative |
| RM13 | negative | negative* | negative |
| RM14 | positive | positive* | positive |
| RM15 | positive | positive | positive |
| RM16 | positive | positive | positive |
| RM17 | positive | negative* | positive |
| RM18 | negative | negative | negative |
| RM19 | negative | negative | negative |
| RM20 | negative | negative | negative |

| | | | |
|---|---|---|---|
| * Manual interpretation. | | | |

Samples M3 and RM1 failed to amplify for pyrosequencing, even after a second extraction; both samples were called V600E-negative (WT) by the TaqMan^{®} and GS20 methods. Four samples required manual interpretation for a pyrosequencing call: For RM17, the pyrosequencing call was V600E-negative whereas both the TaqMan^{®} and GS20 results indicated that the sample was V600E-positive; in the remaining three cases, the three methods gave concordant results. Three additional samples (RM3, RM6, RM8) called V600E-negative by pyrosequencing were V600E-positive by both the TaqMan^{®} and GS20 methods. These results are consistent with an insufficient yield of mutation-containing DNA from RM3, RM6, RM8 and RM17 for mutation detection by pyrosequencing. Sample RM8 was V600E-positive in 4% of the 4300 GS20 Sequencing reads (∼2000 each direction), the lowest percentage of mutated BRAF observed among all of the V600E-positive samples.

Due to the greater sensitivity of GS20 Sequencing compared with pyrosequencing, the GS20 method was used as the reference method for the TaqMan^{®} test. The level of agreement between the COBAS TaqMan^{®} B-Raf V600E Test and GS20 Sequencing is 12/12 = 100% for V600E-positive and 18/18 = 100% for V600E-negative (WT) samples.

### Exemplary Sequences:

**SEQ ID NO:1 BRAF_MU (I = deoxyinosine P= 3'-Phosphate)**
   5' CTACAIAIAAATCTCGATGGAGTGGGTCCCAP 3'
**SEQ ID NO:2 BRAF_WT (I = deoxyinosine P= 3'-Phosphate)**
   5' ACAITGAAATCTCGATGGAGTGGGTCCCAP 3'
**SEQ ID NO:3 BRAF_F1:**
   5' CCTCACAGTAAAAATAGGTGATTTTGGTCT 3'
**SEQ ID NO:4 BRAF_R5:**
   5' TAGCCTCAATTCTTACCATCCACAAAA 3'

## Claims

1. A method of determining sensitivity of cancer cells to a B-Raf kinase inhibitor, the method comprising:
- providing a nucleic acid sample from cancer cells from a patient that has a cancer;
- amplifying a target polynucleotide sequence in the nucleic acid sample using a primer pair that amplifies the target polynucleotide sequence, wherein the target polynucleotide sequence comprises a V600E, a V600D or a V600K mutation site in *BRAF* and amplification is performed in the presence of a labeled oligonucleotide probe that comprises SEQ ID NO: 1, wherein 'n' is deoxyinosine, and detects the presence of a mutated sequence at the V600E, the V600D or the V600K mutation site in *BRAF*; and
- detecting the presence or absence of a V600E, a V600D or a V600K mutation in *BRAF*; thereby determining the sensitivity of the cancer to the B-Raf inhibitor.

2. The method of claim 1, wherein amplification is performed in the presence of a second probe that detects the presence of a wild type sequence at the V600E, the V600D or the V600K mutation site.

3. The method of claim 2, wherein the second probe comprises at least 15 contiguous nucleotides of the nucleotide sequence set forth in SEQ ID NO:2.

4. The method of claim 1, wherein one of the primers in the primer pair comprises at least 15 contiguous nucleotides of the nucleotide sequence set forth in SEQ ID NO:3.

5. The method of claim 1, wherein one of the primers in the primer pair comprises at least 15 contiguous nucleotides of the nucleotide sequence set forth in SEQ ID NO:4.

6. The method of claim 1, wherein the step of amplifying the reaction comprises an RT-PCR.

7. The method of claim 1, wherein the B-Raf kinase inhibitor is a mutant -specific B-Raf kinase inhibitor.

8. The method of claim 1, wherein the B-Raf kinase inhibitor is PLX4032.

9. A kit for detecting a patient that is a candidate for treatment with a B-Raf kinase inhibitor, wherein the kit comprises a first allele-specific probe, wherein the first probe is suitable for detecting a V600E, a V600D or a V600K mutation in *BRAF* and comprises SEQ ID NO: 1, wherein 'n' is deoxyinosine.

10. The kit of claim 9, further comprising a second allele-specific probe, wherein the second probe detects the wild type BRAF sequence and comprises at least 15 contiguous nucleotides of the nucleotide sequence set forth in SEQ ID NO:2.

11. The kit of claim 9, further comprising a primer pair that amplifies a target region of BRAF that comprises a V600E, a V600D or a V600K mutation site.

12. The kit of claim 11, wherein the primer pair comprises a primer that comprises at least 15 contiguous nucleotides of the nucleotide sequence set forth in SEQ ID NO:3.

13. The kit of claim 11, wherein the primer pair comprises primers that have the sequences set forth in SEQ ID NO:3 and SEQ ID NO:4.

14. The kit of claim 9, wherein the first probe has the nucleotide sequence set forth in SEQ ID NO: and the kit further comprises a second probe that has the nucleotide sequence set forth in SEQ ID NO:2, and a primer pair comprises primers that have the nucleotide sequences set forth in SEQ ID NO:3 and SEQ ID NO:4.

## Patentansprüche

1. Verfahren zur Bestimmung der Sensitivität von Krebszellen gegenüber einem B-Raf-Kinase-Hemmer, bei dem man:
- eine Nukleinsäureprobe aus Krebszellen von einem Patienten, der an einer Krebserkrankung leidet, bereitstellt;
- eine Ziel-Polynukleotidsequenz in der Nukleinsäureprobe unter Verwendung eines Primerpaars, mit dem die Ziel-Polynukleotidsequenz amplifiziert wird, amplifiziert, wobei die ziel-Polynukleotidsequenz eine V600E-, eine V600D- oder eine V600K-Mutationsstelle in *BRAF* umfasst und die Amplifikation in Gegenwart einer markierten Oligonukleotidsonde erfolgt, die SEQ ID NO:1 umfasst, wobei 'n' für Desoxyinosin steht, und mit der das Vorliegen einer mutierten Sequenz an der V600E-, der V600D- oder der V600K-Mutationsstelle in *BRAF* nachgewiesen wird; und
- das Vorliegen oder Fehlen einer V600E-, einer V600D- oder einer V600K-Mutation in *BRAF* nachweist; wodurch die Sensitivität des Krebsgeschwürs gegenüber dem B-Raf-Hemmer bestimmt wird.

2. Verfahren nach Anspruch 1, wobei die Amplifikation in Gegenwart einer zweiten Sonde erfolgt, mit der das Vorliegen einer Wildtyp-Sequenz an der V600E-, der V600D- oder der V600K-Mutationsstelle nachgewiesen wird.

3. Verfahren nach Anspruch 2, wobei die zweite Sonde wenigstens 15 zusammenhängende Nukleotide der Nukleotidsequenz gemäß SEQ ID NO:2 umfasst.

4. Verfahren nach Anspruch 1, wobei einer der Primer in dem Primerpaar wenigstens 15 zusammenhängende Nukleotide der Nukleotidsequenz gemäß SEQ ID NO:3 umfasst.

5. Verfahren nach Anspruch 1, wobei einer der Primer in dem Primerpaar wenigstens 15 zusammenhängende Nukleotide der Nukleotidsequenz gemäß SEQ ID NO:4 umfasst.

6. Verfahren nach Anspruch 1, wobei der Schritt des Amplfizierens der Reaktion eine RT-PCR umfasst.

7. Verfahren nach Anspruch 1, wobei es sich bei dem B-Raf-Kinase-Hemmer um einen mutantenspezifischen B-Raf-Kinase-Hemmer handelt.

8. Verfahren nach Anspruch 1, wobei es sich bei dem B-Raf-Kinase-Hemmer um PLX4032 handelt.

9. Kit zum Identifizieren eines Patienten, der ein Kandidat für die Behandlung mit einem B-Raf-Kinase-Hemmer ist, wobei das Kit eine erste allelspezifische Sonde umfasst, wobei die erste Sonde zum Nachweisen einer V600E-, einer V600D- oder einer V600K-Mutation in *BRAF* geeignet ist und SEQ ID NO:1 umfasst, wobei 'n' für Desoxyinosin steht.

10. Kit nach Anspruch 9, ferner umfassend eine zweite allelspezifische Sonde, wobei mit der zweiten Sonde die Wildtyp-BRAF-Sequenz nachgewiesen wird und die zweite Sonde wenigstens 15 zusammenhängende Nukleotide der Nukleotidsequenz gemäß SEQ ID NO:2 umfasst.

11. Kit nach Anspruch 9, ferner umfassend ein Primerpaar, mit dem eine Zielregion von BRAF, die eine V600E-, eine V600D- oder eine V600K-Mutationsstelle umfasst, amplifiziert wird.

12. Kit nach Anspruch 11, wobei das Primerpaar einen Primer umfasst, der wenigstens 15 zusammenhängende Nukleotide der Nukleotidsequenz gemäß SEQ ID NO:3 umfasst.

13. Kit nach Anspruch 11, wobei das Primerpaar Primer umfasst, die die Sequenzen gemäß SEQ ID NO:3 und SEQ ID NO:4 aufweisen.

14. Kit nach Anspruch 9, wobei die erste Sonde die Nukleotidsequenz gemäß SEQ ID NO:1 aufweist und das Kit ferner eine zweite Sonde, die die Nukleotidsequenz gemäß SEQ ID NO:2 aufweist, sowie ein Primerpaar, das Primer umfasst, die die Nukleotidsequenzen gemäß SEQ ID NO:3 und SEQ ID NO:4 aufweisen, umfasst.

## Revendications

1. Procédé de détermination de la sensibilité de cellules cancéreuses à un inhibiteur de B-Raf kinase, le procédé comprenant :
- la fourniture d'un échantillon d'acide nucléique de cellules cancéreuses d'un patient qui a un cancer ;
- l'amplification d'une séquence polynucléotidique cible dans l'échantillon d'acide nucléique, en utilisant une paire d'amorces qui amplifie la séquence polynucléotidique cible, dans lequel la séquence polynucléotidique cible comprend un site de mutation V600E, V600D ou V600K dans BRAF et l'amplification est réalisée en présence d'une sonde oligonucléotidique marquée qui comprend SEQ ID n°1, dans laquelle « n » est la désoxyinosine, et détecte la présence d'une séquence mutée au site de mutation V600E, V600D ou V600K dans BRAF ; et
- la détection de la présence ou de l'absence d'une mutation V600E, V600D ou V600K dans BRAF ; déterminant ainsi la sensibilité du cancer à l'inhibiteur de B-Raf.

2. Procédé selon la revendication 1, dans lequel l'amplification est réalisée en présence d'une seconde sonde qui détecte la présence dune séquence sauvage au site de mutation V600E, V600D ou V600K.

3. Procédé selon la revendication 2, dans lequel la seconde sonde comprend au moins 15 nucléotides contigus de la séquence nucléotidique présentée dans SEQ ID n° 2.

4. Procédé selon la revendication 1, dans lequel une des amorces dans la paire d'amorces comprend au moins 15 nucléotides contigus de la séquence nucléotidique présentée dans SEQ ID n° 3.

5. Procédé selon la revendication 1, dans lequel une des amorces dans la paire d'amorces comprend au moins 15 nucléotides contigus de la séquence nucléotidique présentée dans SEQ ID n° 4.

6. Procédé selon la revendication 1, dans lequel dans l'étape d'amplification la réaction comprend une RT-PCR.

7. Procédé selon la revendication 1, dans lequel l'inhibiteur de B-Raf kinase est un inhibiteur de B-Raf kinase spécifique de mutant.

8. Procédé selon la revendication 1, dans lequel l'inhibiteur de B-Raf kinase est le PLX4032.

9. Kit de détection d'un patient qui est un candidat pour le traitement avec un inhibiteur de B-Raf kinase, dans lequel le kit comprend une première sonde spécifique d'allèle, dans lequel la première sonde est adaptée pour détecter une mutation V600E, V600D ou V600K dans BRAF et comprend SEQ ID n° 1, dans laquelle « n » est la désoxyinosine.

10. Kit selon la revendication 9, comprenant en outre une seconde sonde spécifique d'allèle, dans lequel la seconde sonde détecte la séquence de BRAF sauvage et comprend au moins 15 nucléotides contigus de la séquence nucléotidique présentée dans SEQ ID n° 2.

11. Kit selon la revendication 9, comprenant en outre une paire d'amorces qui amplifie une région cible de BRAF qui comprend un site de mutation V600E, V600D ou V600K.

12. Kit selon la revendication 11, dans lequel la paire d'amorces comprend une amorce qui comprend au moins 15 nucléotides contigus de la séquence nucléotidique présentée dans SEQ ID n° 3.

13. Kit selon la revendication 11, dans lequel la paire d'amorces comprend des amorces qui ont les séquences présentées dans SEQ ID n° 3 et SEQ ID n° 4.

14. Kit selon la revendication 9, dans lequel la première sonde a la séquence nucléotidique présentée dans SEQ ID n° 1 et le kit comprend en outre une seconde sonde qui a la séquence nucléotidique présentée dans SEQ ID n° 2, et une paire d'amorces comprend les amorces qui ont les séquences nucléotidiques présentées dans SEQ ID n° 3 et SEQ ID n° 4.
